# EUROPÄISCHE PATENTSCHRIFT

(11) **EP 3 481 180 B1**
(45) Veröffentlichungstag und Bekanntmachung des Hinweises auf die Patenterteilung: **07.06.2023**
(21) Anmeldenummer: 17743267.1
(22) Anmeldetag: 07.07.2017
(51) Int. Cl.: A01K 1/03, A01H 4/00

(54) **KARUSSELLISOLATOR**
CAROUSEL ISOLATOR
CARROUSEL ISOLATEUR

(30) Priorität: 08.07.2016 DE 102016112549
(43) Veröffentlichungstag der Anmeldung: 15.05.2019
(73) Patentinhaber: Hammelbacher, Stephan, 83684 Tegernsee (DE)
(72) Erfinder: Hammelbacher, Stephan, 83684 Tegernsee (DE)
(74) Vertreter: Beckord & Niedlich Patentanwälte PartG mbB
(86) Internationale Anmeldenummer: PCT/EP2017/067113
(87) Internationale Veröffentlichungsnummer: WO 2018/007596

(56) Entgegenhaltungen:
- EP-A2- 2 492 663
- EP-A2- 2 805 609
- DE-A1- 1 507 065
- US-A- 3 302 615
- US-A- 3 877 420
- US-A- 5 194 041

## Beschreibung

Die Erfindung betrifft eine Isolatoranordnung zum Herstellen und zum Aufrechterhalten einer keimfreien Umgebung auch unter Arbeitsbedingungen, insbesondere für die Pflanzenzucht, für die Tierforschung oder zur Vorbereitung von Medikamenten. Sie dient zur Aufnahme einer Vielzahl von Behältern, die jeweils mindestens ein Objekt enthalten. Die Objekte sollen zu Forschungszwecken unter möglichst klar definierten Bedingungen gehalten und ggf. manipuliert werden. Dazu dient eine Arbeitsplatte, auf der mindestens zwei Behälter abgestellt und an ihnen oder ihrem Inhalt Leistungen erbracht oder Vorgänge vorgenommen werden können. Auch dabei sollen die definierten Bedingungen nicht beeinträchtigt werden. Dafür verfügt die Isolatoranordnung über eine Schleuseneinrichtung, um ohne Beeinträchtigung der definierten Bedingungen Material in den Isolator ein- und aus ihm ausschleusen zu können. Die Bedienung des Isolators soll überdies ergonomischen Anforderungen eines Bedieners gerecht werden.

Eine Isolatoranordnung für die Aufnahme und die Bearbeitung einer Vielzahl von Behältern zeigt die US 33 02 615 A. Sie verfügt über mehrere bewegbare Regalanordnungen zur Aufnahme der Behälter, denen jeweils eine Arbeitsplatte zugeordnet ist. Mehrere Regalanordnungen verfügen über eine gemeinsame Schleuseneinrichtung und erfordern daher einen gewissen Platzbedarf.

Die EP 2 805 609 A2 betrifft ein Tierzuchtsystem, das ein Gestell, einen Trägerroboter, eine Wärmebildkamera und eine Anweisungseinheit beinhaltet. Das Gestell enthält darin einen Käfig zur Zucht eines Tieres. Der Trägerroboter beinhaltet eine Hand mit einem Haltemechanismus für den Käfig. Die Wärmebildkamera wird in der Nähe der Hand bereitgestellt. Die Anweisungseinheit weist den Trägerroboter an, einen Vorgang durchzuführen, bei dem der Käfig in und aus dem Gestell ein- und ausgefahren wird, während der Käfig mit der Hand gehalten wird, um den Käfig zwischen dem Gestell und einer bestimmten Trageposition zu tragen, und einen Vorgang, bei dem die Wärmebildkamera nahe an den Käfig im Gestell gebracht wird, um zu bewirken, dass die Wärmebildkamera das Innere des Käfigs erfasst.

Aufgabe der Erfindung ist es daher, eine Isolatoranordnung anzugeben, die bei einem möglichst geringen Raumbedarf ergonomische Anforderungen eines Benutzers erfüllt.

Diese Aufgabe wird durch eine Isolatoranordnung, insbesondere für die Pflanzenzucht oder die Tierforschung, erfüllt, die folgende Bestandteile aufweist:
- ein zylindrisches Regal, das aufgrund seiner geometrischen Gestalt über eine vertikale Hochachse verfügt,
- eine Vielzahl an Behältern zur Aufnahme jeweils mindestens eines Objekts als deren Inhalt, die im Regal umfangseitig angeordnet und in einer radialen Richtung zugänglich bzw. bedienbar sind,
- eine Arbeitsplatte in unmittelbarer räumlicher Nähe zu dem Regal, also in einer Entfernung von maximal einer Armlänge,
- ein Zelt bzw. eine Hülle zur gemeinsamen sterilen, luftdichten Abschirmung des Regals und zugleich der Arbeitsplatte gegenüber der Umgebung,
- Handschuheingriffe im Zelt zur Bedienung und zur Bearbeitung der Behälter und ihres Inhalts zwischen dem Regal und der Arbeitsplatte bzw. auf der Arbeitsplatte,
- eine Schleuseneinrichtung im Zelt zum Einbringen sterilen Materials in das Zelt und zum Entfernen kontaminierten Materials aus dem Zelt, und
- eine Vorrichtung zur Verlagerung des Regals relativ zur Arbeitsplatte in einer axialen bzw. vertikalen Richtung, also entlang seiner Hochachse, und in einer rotatorischen Richtung, also um seine Hochachse herum.

Erfindungsgemäß verfügt die Isolatoranordnung also über ein in seiner äußeren Gestalt im Prinzip kreiszylinderförmiges Regal mit mehreren horizontal übereinander angeordneten Etagen, das um seine Hochachse herum im Wesentlichen kreissektorförmige Behälter aufnehmen kann. Mit ebenflächigen statt gebogenen Frontseiten der Behälter kann das Regal tatsächlich über einen vieleckigen Grundriss verfügen, bei sechs Behältern je Etage beispielsweise über einen hexagonalen Grundriss. Die Behälter lassen sich in einer radialen Richtung in das Regal einschieben und in der Gegenrichtung entnehmen. Den prinzipiellen Aufbau eines derartigen kreiszylinderförmigen Regals zeigt beispielsweise die DE 1 507 065 A.

Zur Bearbeitung der Behälter und/oder ihres Inhalts verfügt die Isolatoranordnung über eine Arbeitsplatte, die vom Regal nicht weiter entfernt ist als in etwa eine Armlänge. Damit kann ein Benutzer, der vor der Arbeitsplatte steht, über sie hinweg einen Behälter aus dem Regal entnehmen und bestimmungsgemäß bearbeiten. Damit der Benutzer bequem vor der Arbeitsplatte stehen oder ggf. sitzen kann, kann die Isolatoranordnung unter der Arbeitsplatte einen Fußbereich freilassen, dessen Fläche im Wesentlichen der horizontalen Ausdehnung der Arbeitsplatte entspricht.

Sowohl die Arbeitsplatte als auch das Regal sind gemeinsam von einem Zelt bzw. von einer Hülle umschlossen, das bzw. die eine gasdichte und damit sterile Abschirmung gegenüber der Umgebung bildet. Die Begriffe Zelt und Hülle sind funktional zu verstehen und schließen sowohl eine flexible bzw. verformbare Abschirmung wie auch eine solche aus einem steifen Material sowie Kombinationen aus steifen und flexiblen Abschnitten ein. Das Material des Zelts lässt sich neben seiner Eigenschaft als flexibel oder starr auch nach weiteren Kriterien wie einer möglichst guten Transparenz und seiner Dauerhaftigkeit gegenüber den eingesetzten Sterilisierungsmitteln auswählen. Die Transparenz dient einer guten Licht- und Sichtdurchlässigkeit, sodass das Regal selbst keine eigene Lichtquelle erfordert und durch das Zelt hindurch behinderungsfrei in Augenschein genommen werden kann. Die Dauerhaftigkeit gegenüber den Sterilisierungsmitteln bestimmt maßgeblich die Lebensdauer des Zelts. Je nach Anforderung und gewählter Sterilisierungsmethode können mögliche Materialien für das Zelt PVC (Polyvinylchlorid), Vinyl, Polyurethan oder Acryl, PSU (Polysulfon), PC (Polycarbonat) oder PEI (Polyetherimid) sein.

Damit nun ein Bediener die Behälter bzw. deren Inhalt manipulieren kann, ohne selbst vollständig in das Zelt eintreten zu müssen, verfügt die Isolatoranordnung über Handschuheingriffe, die über der Arbeitsplatte derart angeordnet sind, dass sie einen Zugriff auf Behälter im Regal und Tätigkeiten auf der Arbeitsplatte ermöglichen.

Um auch einen Materialaustausch zwischen dem Innenraum des Zelts, also im Wesentlichen zwischen dem Regal, und der ihm gegenüber abgeschirmten Umgebung zu ermöglichen, verfügt die Isolatoranordnung über eine an sich bekannte Schleuseneinrichtung. Damit lassen sich zumindest einzelne Behälter durch das Zelt hindurch transportieren, ohne dass die definierten Bedingungen innerhalb des Zelts beeinträchtigt würden.

Schließlich verfügt die Isolatoranordnung über eine oder ggf. jeweils eine Vorrichtung zur Verlagerung des Regals relativ zur Arbeitsplatte, und zwar in einer axialen Richtung des Regals, also in einer vertikalen Richtung aufwärts und abwärts, und außerdem zur Verlagerung in einer rotatorischen Richtung des Regals um die Hochachse herum, also zur Drehung des im Wesentlichen zylindrischen Regals um seine Hoch- bzw. Zylinderachse. Die Drehung um seine Zylinderachse relativ zur Arbeitsplatte ermöglicht die Zugänglichkeit des Regals von derselben Position der Arbeitsplatte aus an jeden Punkt des Umfangs des Regals. Durch Drehen des Regals relativ zur Arbeitsplatte lassen sich also von der Arbeitsplatte aus grundsätzlich alle Behälter des Regals erreichen, unabhängig davon, an welcher Stelle in Umfangsrichtung betrachtet sie positioniert sind.

Für die rotatorische Relativbewegung lassen sich prinzipiell drei Ausführungsformen der Isolatoranordnung unterscheiden:
- das Regal kann um seine Hochachse drehbar gelagert und die Arbeitsplatte feststehend angeordnet sein, oder
- die Arbeitsplatte kann rotatorisch um die Hochachse des Regals schwenkbar und das Regal selbst feststehend angeordnet sein, oder
- sowohl das Regal als auch die Arbeitsplatte können um die Hochachse des Regals drehbar bzw. schwenkbar gelagert und beide damit beweglich gegenüber einem stationären Widerlager angeordnet sein.

Jede dieser drei Varianten hat ihre spezifischen Vorteile, die im jeweiligen Anwendungsfall im Vordergrund stehen können. So unterliegen die Behälter eines stationären Regals und einer schwenkbaren Arbeitsplatte keinen Bewegungen, was bei Tierforschungseinrichtungen mit mit Tieren besetzten Käfigen als Behältern von Vorteil sein kann. Eine stationäre Arbeitsplatte mit einem drehbaren Regal dagegen kann zu einem sehr kompakten Aufbau der Isolatoranordnung führen, was gerade in kleineren Forschungseinrichtungen den Einsatz der Isolatoranordnung überhaupt erst ermöglichen kann. Ein sowohl drehbares Regal als auch eine schwenkbare Arbeitsplatte wiederum können richtungsmäßig gegeneinander bewegt und so zu einer besonders schnellen Betätigung der Isolatoranordnung durch einen schnellen Zugriff auf die Behälter führen, was die Arbeitsabläufe innerhalb der Isolatoranordnung verkürzen kann.

Die vertikale Relativbewegung des Regals und der Arbeitsplatte zueinander ermöglicht ein umfangreicheres Regal in seiner Höhenerstreckung, indem seine Vertikalbewegung relativ zur Arbeitsplatte die Zugänglichkeit auch hoch- oder tiefliegender Behälter im Regal trotz der eingeschränkten Bewegungsmöglichkeiten eines Benutzers durch die Handschuheingriffe hindurch zulässt.

Auch die Vertikalbewegung des Regals relativ zur Arbeitsplatte lässt sich in der für die Rotation beschriebenen Weise nach drei grundsätzlichen Prinzipien ausbilden:
- das Regal kann gegenüber der feststehenden Arbeitsplatte vertikal verschiebbar sein,
- die Arbeitsplatte kann vertikal gegenüber dem feststehenden Regal verschiebbar sein und schließlich
- können sowohl das Regal als auch die Arbeitsplatte unabhängig voneinander gegenüber einem stationären Widerlager vertikal verschiebbar sein.

Diese Konstruktionen bieten prinzipiell die entsprechenden Vorteile wie bei den drei rotatorischen Bewegunmöglichkeiten angegeben.

Weitere Variationsmöglichkeiten ergeben sich dadurch, dass das Regal grundsätzlich beweglich und die Arbeitsplatte grundsätzlich stationär oder umgekehrt ausgebildet sein können. Darüber hinaus kann das Regal beispielsweise vertikal beweglich, aber rotatorisch stationär und die Arbeitsplatte rotatorisch beweglich und vertikal stationär ausgebildet sein oder umgekehrt. Vorzüge der jeweiligen Ausführungsformen können beispielsweise im Bedienungskomfort oder in der konstruktiven Einfachheit der Vorrichtungen zur rotatorischen oder translatorischen Bewegung des Regals oder der Arbeitsplatte oder beider liegen. Ihre Auswahl kann insbesondere daran orientiert sein, welche Bewegungsanforderungen zu erfüllen sind und sich unter den Bedingungen eines hermetisch abschirmenden Zelts am günstigsten ausbilden lassen.

Insbesondere in einer Ausgestaltungsform der Isolatoranordnung mit einem lediglich drehbaren, vertikal aber stationären Regal kann die Isolatoranordnung an einer nur vertikal bewegbaren Arbeitsplatte ein Roboterarm zur Entnahme der Behälter aus dem Regal und zum Abstellen auf der Arbeitsplatte umfassen. Der Roboterarm kann sich mit der Arbeitsplatte zusammen vor dem drehbaren Regal vertikal verfahren lassen und dem Benutzer die Entnahme der Behälter aus dem Regal abnehmen. Damit entlastet er den Benutzern nicht nur von einer körperlich anstrengenden Tätigkeit, sondern das Regal kann auch erheblich höher ausgeführt werden, weil es nicht mehr auf eine Entnahme von Behältern durch den Benutzer mithilfe der Handschuheingriffe ausgelegt werden muss, die den Benutzer in seinen Bewegungsmöglichkeiten und insbesondere in seiner Reichweite einschränken. Auf derselben Grundfläche lassen sich damit erheblich mehr Behälter unterbringen, was zu einem wirtschaftlichen Betrieb der Isolatoranordnung beitragen kann.

Gemeinsam ist jedenfalls allen Ausführungsformen der Isolatoranordnung mit einem zylindrischen Regal und einer Arbeitsplatte, die sowohl rotatorisch als auch translatorisch in einer linearen Bewegungsrichtung gegeneinander verstellbar sind, dass sie trotz zwingender Bedienung per Handschuheingriffen eine ergonomisch günstige Zugänglichkeit einer Vielzahl an Behältern in einem hermetisch abschirmenden Zelt bieten.

Eine rotatorische Verlagerung des Regals gegenüber einer Arbeitsplatte kann bei einer geeigneten leichtgängigen Lagerung per Muskelkraft erfolgen. Vertikal ausgerichtete Griffe am Umfang des Regals können eine händische Drehbewegung des Regals erleichtern, ein Abstoßen des Benutzers am Boden kann ein Verschwenken einer schwenkbar gelagerten Arbeitsplatte um ein feststehendes Regal herum ermöglichen. Eine vertikale Verlagerung erfordert wegen des Gewichts des Regals oder der Arbeitsplatte samt Hülle in der Regel eine mechanische Unterstützung des Benutzers. Sie kann beispielswese in vorgespannten oder in Gasdruck-Federn bestehen und eine Pedalbetätigung umfassen. Nach einer vorteilhaften Ausgestaltung der Erfindung kann die Isolatoranordnung zumindest einen motorischen Antrieb zur Verlagerung des Regals relativ zur Arbeitsplatte aufweisen. Der motorische Antrieb erleichtert die Verlagerung grundsätzlich, kann aber bei seiner Anordnung innerhalb des Zelts konstruktive Herausforderungen bieten. Er kann daher vorzugsweise außerhalb des Zelts angeordnet sein. Dort bietet er eine leichtere Zugänglichkeit für Wartungs- und eventuelle Austauscharbeiten und schließt Verschmutzungen beispielsweise durch den Abrieb von Kohlebürsten eines Elektromotors im Zelt aus. Dieser Aspekt betrifft vor allem den motorische Antrieb zur Verlagerung des Regals, sofern er weitgehend vollständig innerhalb des Zelts angeordnet ist, wohingegen ein Antrieb der Arbeitsplatte - die günstiger Weise mit einer Bewegung des Zelts um das Regal herum oder an ihm auf und ab zusammenfällt - vorteilhaft von einer Außenseite der Isolatoranordnung aus und damit weitgehend konventionell erfolgen kann. Die Steuerung des Motors oder der Motoren kann entweder über Servomotoren, die Soft-Start- und Soft-Stopfunktionen und die komplette Steuerungslogik beinhalten oder über auf Klemmleiste gelegte Kabel und einen hier angeschlossenen Kontroller mit Maschinensteuerung erfolgen.

Grundsätzlich kann die rotatorische Verlagerung des Regals gegenüber der feststehenden Arbeitsplatte durch eine Drehung des gesamten Regals erfolgen. Nach einer vorteilhaften Ausgestaltung der Erfindung kann das Regal einzelne horizontal verlaufende scheibenförmige Tellern umfassen, auf denen sich die Behälter abstellen lassen. Die Teller müssen nicht geschlossenflächig ausgebildet sein, sondern können Gitter oder Tragrahmen zum schienengeführten Einschieben der Behälter aufweisen. Vorteilhafterweise können lediglich einzelne Teller unabhängig von einer Drehung des gesamten Regals separat um die Hochachse gedreht werden. Ein einzelner Teller kann also gegenüber einem ruhenden Regal oder gegenüber einem sich drehenden Regal individuell verdreht werden, um einen schnelleren Zugriff des Benutzers auf einen gewünschten Behälter zu erreichen. Das individuelle Verdrehen einzelner Teller spart Energie, weil nicht das gesamte Regal in Bewegung versetzt werden muss. Außerdem kann es den Zugriff auf einen gewünschten Behälter beschleunigen, weil bei der Verdrehung eines Tellers eine geringere Massenträgheit überwunden werden muss als bei der Drehung des gesamten Regals. Eine besonders kostengünstige Variante kann daher eine Isolatoranordnung mit einem ausschließlich vertikal bewegbaren Regal darstellen, wobei die Arbeitsplatte stationär angeordnet ist und dessen Behälter durch manuelles Drehen nur einzelner Regalteller zu erreichen sind. Damit kann ein motorischer Antrieb der Rotation des Regals und dessen Kapselung oder Abschirmung gegenüber der Atmosphäre innerhalb des Zelts entfallen, was auch den Unterhalt der Isolatoranordnung durch Entfall von Wartungs- und Reparaturarbeiten vergünstigt.

Eine konstruktive Herausforderung kann insbesondere die vertikale Verfahrbarkeit des Regals innerhalb des Zelts darstellen. So kann die Vertikalbewegung mittels einer in Richtung der Hochachse verlaufenden Zahnstange und eines damit kämmenden Zahnrads in eine Drehbewegung umgesetzt werden, wobei die Drehbewegung über eine Achse durch das Zelt hindurch nach außen geführt werden kann. Die drehbare Achse lässt sich gegenüber dem feststehenden Zelt relativ zuverlässig in einer geeigneten Durchführung luftdicht abdichten. Alternativ kann eine hydraulische Hubvorrichtung angeordnet sein, die allerdings externe Ölwannen benötigen kann. Zudem stellt eine ggf. undichte Hydraulik ein hohes Risiko in der Isolatoranordnung dar.

Nach einer weiteren vorteilhaften Ausgestaltung der Erfindung kann das Regal über einen Scherenhubantrieb vertikal verstellbar sein, dessen Antrieb ebenfalls durch eine rotatorische oder translatorische Bewegung und deren Durchführung durch das Zelt hindurch zuverlässig abdichtbar sein kann. Mit einer geeigneten Dimensionierung der Scheren des Hubantriebs kann er geeignet flach aufgebaut werden, um möglichst wenig Raum unterhalb des Regals zu beanspruchen.

Alternativ dazu kann die Isolatoranordnung über einen teleskopierbaren Spindelhubantrieb zur axialen bzw. vertikalen Verlagerung des Regals verfügen. Der Spindelhubantrieb kann zusammen mit einer teleskopierbaren Hubsäule sehr kompakt ausgebildet werden, wie beispielsweise unter www.spindelhubgetriebe.com der Firma IFIA zu ersehen. Eine Alternative dazu kann ein Spirallift beispielsweise der Firma PACO (www.pacospiralift.com) darstellen, der ebenfalls in einer eingefahrenen Position eine geringe Bauhöhe aufweist und dennoch einen beträchtlichen Hub ermöglicht. Die teleskopierbaren Hubsäulen beider Hubeinrichtungen können mit einem Faltenbalg, nämlich einem elastischen, sich "ziehharmonikaartig" zusammenfaltenden Schlauch aus Gummi oder Kunststoff, luftdicht abgeschlossen gegenüber der Atmosphäre des Zelts dorthin einfahren lassen, so dass der Antrieb vollkommen außerhalb des Zelts verbleiben kann. Die ggf. zusätzlich im Balg abzudichtende Drehbewegung des Regals kann durch an sich bekannte Isolationstechniken im Isolatorbau, z.B. durch Abdichtung mit Hilfe von Silikon, bewerkstelligt werden.

Grundsätzlich lassen sich die Vorrichtungen für die rotatorische und die vertikale translatorische Bewegung des Regals relativ zur Arbeitsplatte nach zwei unterschiedlichen Prinzipien relativ zueinander anordnen: die rotatorische Vorrichtung kann unterhalb der translatorischen Vorrichtung angebracht sein, so dass die translatorische Vorrichtung der Bewegung der rotatorischen Vorrichtung folgt. Alternativ kann die Rotationsvorrichtung auf der Translationsvorrichtung angeordnet sein, so dass jene einer rotatorischen Bewegung nicht folgen muss, sondern die Rotationsvorrichtung eine translatorische Bewegung mitmacht. In beiden Fällen ist in der Regel die Energieversorgung derjenigen Vorrichtung konstruktiv aufwändiger, die der Bewegung der anderen Vorrichtung folgt. Vorteilhafterweise verfügt die Isolatoranordnung über eine drehbare Vorrichtung zur axialen Verlagerung des Regals relativ zur feststehenden Arbeitsplatte. Dazu kann die Hubvorrichtung beispielsweise auf einer Drehscheibe angeordnet sein, die ihren - mechanischen, insbesondere muskelbetriebenen, oder zum Beispiel elektrischen - Antrieb außerhalb des Zelts findet und sich durch eine sterile und luftdichte Durchführung im Zelt in Rotation versetzen lässt. Diese Anordnung erfordert zum Beispiel eine absolut dichte Hubsäule, um Kontaminationen des Innenraums des Isolators aufgrund der Hubeinrichtung auszuschließen. Alternativ kann ein flexibler und hermetisch dichter Faltenbalg die Hubsäule oder einen Scherenhubantrieb umgeben. Der Balg kann damit einen Teil der Hülle darstellen, der die translatorische Bewegung der Hubvorrichtung und des Regals mitmacht. Eine Drehung des Regals kann eine Rotationsdichtung des Balgs an einer Mantelfläche der Hubvorrichtung oder des Regals ermöglichen.

Grundsätzlich kann das Zelt bzw. die Hülle der Isolatoranordnung einteilig bzw. einstückig ausgebildet sein, was die Gefahr von Komplikationen und Undichtigkeiten reduziert. Nach einer vorteilhaften Ausgestaltung der Isolatoranordnung kann die Hülle jedoch mehrteilig aus miteinander koppelbaren Abschnitten aufgebaut sein. Dazu können die Abschnitte über Kopplungsvorrichtungen zur luftdichten Befestigung aneinander verfügen. Vorzugsweise können die Kopplungsvorrichtungen lösbar ausgebildet sein, um eine beschädigungsfreie Demontage der Hülle beispielsweise zu Reinigungsarbeiten zu ermöglichen. Eine einfache Kopplungsvorrichtung sieht eine Nut- und Federausbildung an den einander zugewandten Stirnseiten der Abschnitte vor. Sie stellt eine einfach herzustellende und zu montierende Vorrichtung dar, die weitgehend luftdicht abschließt. Die verbleibenden Fugen können nach dem Koppeln der Abschnitte sicherheitshalber mit Silikon abgedichtet oder mit einer transparenten Folie vollflächig abgeklebt werden.

Eine mehrteilige Hülle bietet darüber hinaus die Möglichkeit einer Erweiterung, so dass beispielsweise bei einer Vergrößerung des Regals zur Aufnahme zusätzlicher Behälter die Hülle durch Einfügen eines Erweiterungsabschnitts angepasst werden kann. Damit kann der technische und finanzielle Aufwand für eine neue Hülle entfallen. Geeignete Abschnitte der erfindungsgemäßen Hülle können beispielsweise in einem Bodenabschnitt, einem Abschnitt mit Handschuheingriffen, einem kreiszylindrischen Abschnitt und einem Deckelabschnitt bestehen. Durch Weglassen oder Einfügen des kreiszylindrischen Abschnitts oder durch Einfügen mehrerer kreiszylindrischer Abschnitte kann sich die Isolatoranordnung an unterschiedlich hohe Regale mit geringem Aufwand anpassen lassen. Auch der Austausch einzelner Abschnitte, beispielsweise aufgrund von Defekten, kann zu einer besonders wirtschaftlichen Betriebsweise der Isolatoranordnung führen.

Nach einer weiteren vorteilhaften Ausgestaltung der Erfindung kann die Isolatoranordnung über eine Steuerungseinrichtung zur Steuerung der vertikalen und der Drehbewegung des Regals relativ zur Arbeitsplatte und insbesondere zur Steuerung der Drehung einzelner Regalteller verfügen. Die Steuerungseinrichtung kann alternativ oder zusätzlich zur Steuerung von Vorgängen mit Behältern und/oder Leistungen an Objekten in den Behältern dienen und eine Datenbank zur Erfassung der Objekte und zur Dokumentation von Vorgängen mit und Leistungen an Behältern und/oder deren Objekten aufweisen. Damit kann eine erhebliche Erleichterung der Bedienung der Isolatoranordnung an sich und eine Dokumentation und Fehlervermeidung der Tätigkeiten an der Isolatoranordnung einhergehen. Eine geeignete Steuerung ist in der WO 2011/124 209 A desselben Anmelders detailliert angegeben, deren diesbezüglicher Inhalt auch zum Inhalt der vorliegenden Anmeldung gemacht wird.

Nach einer weiteren vorteilhaften Ausgestaltung der Erfindung kann die Isolatoranordnung über Kennzeichnungen der Behälter zu deren Individualisierung und über korrespondierende Leseeinrichtungen verfügen. Eine geeignete Technologie beschreibt die WO 2014/053 436 A desselben Anmelders, deren diesbezüglicher Inhalt auch zum Offenbarungsgehalt der vorliegenden Anmeldung gemacht wird. Mit einer transparenten Hülle bietet sich die Möglichkeit, die Leseeinrichtungen außerhalb der Hülle anzuordnen und durch sie hindurch wirken zu lassen, so dass der Aufwand für deren Autoklavierung vor dem Einbau und jeder ggf. erforderlichen Reparatur oder Wartung entfallen kann. Alternativ oder zusätzlich können die Behälter mit so genannten "CageTalkern" oder RFID bestückt über NFC- oder RFID-Lesegeräte, die außerhalb der Hülle und dort zum Beispiel in der Mitte unterhalb der Arbeitsplatte angebracht sind, automatisch gelesen werden. Dies erlaubt ein softwareseitiges "automatisches Öffnen" gezogener Behälter in einer Steuerungssoftware, wie in der WO 2011/124 209 A grundsätzlich beschrieben.

Mit Hilfe von zum Beispiel drei Kameras, die um das Regal herum angeordnet sind, oder mit zwei 3D-Kameras lässt sich feststellen, aus welchem Fach des Regals ein Behälter gezogen wurde. Diese Information kann an die Steuerungssoftware weitergegeben werden, wie im Einzelnen auch in der WO 2014/053 436 A beschrieben. Außerdem kann eine Kamera direkt am Zelt fixiert beispielsweise das Verhalten von Tieren in einzelnen Behältern beobachten. Über die Steuerungssoftware kann sich das Regal in vorgegebenen Intervallen drehen und heben und senken, um alle Behälter zu erfassen. Alternativ können mehrere Kameras installiert sein. Ein hochtransparentes Zelt erlaubt die Montage der Kameras außerhalb des Zelts

Eine Schleuseneinrichtung kann an weitgehend jeder beliebigen und geeigneten Stelle, zum Beispiel seitlich am Zelt angebracht sein. Zu Bedienungszwecken, nämlich zum Be- und Entladen der Schleuseneinrichtung, sind weitere Handschuheingriffe erforderlich. Nach einer weiteren vorteilhaften Ausgestaltung der Erfindung kann die Isolatoranordnung über eine in der Arbeitsplatte integrierte Schleuseneinrichtung verfügen. Zur Anordnung der Schleuseneinrichtung kann die Isolatoranordnung unter der Arbeitsplatte einen Fußraum frei lassen, der etwa der horizontalen Ausdehnung der Arbeitsplatte entspricht und der für Sicherheitsbehälter verwendet werden kann, die an der Schleuseneinrichtung ankoppelbar sind. Die Sicherheitsbehälter lassen sich folglich von einer Unterseite der Arbeitsplatte aus an der Isolatoranordnung ankoppeln. Damit lässt sich ein besonders kompakter Aufbau erreichen, weil der Raum unter der gegenüber dem zylindrischen Regal auskragenden Arbeitsplatte anderweitig kaum vollständig genutzt werden kann. Außerdem liegt die Schleuseneinrichtung damit im unmittelbaren Zugriffsbereich der für die Bedienung der Arbeitsplatte ohnehin erforderlichen Handschuheingriffe, so dass die Schleuseneinrichtung vom Standort des Benutzers aus bequem betätigt werden kann. Der konstruktive Aufwand und der Platzbedarf für weitere Handschuheingriffe allein zur Bedienung der Schleuseneinrichtung kann damit vorteilhaft eingespart werden.

Bei Nichtgebrauch der Schleuseneinrichtung können anstelle der Sicherheitsbehälter Blindbehälter geringerer Ausdehnung und allein zu Abdichtungszwecken montiert werden. Dann kann der volle Fußraum für den Benutzer der Isolatoranordnung zur Verfügung stehen.

Nach einer weiteren vorteilhaften Ausgestaltung der Erfindung kann die Schleuseneinrichtung über zwei Öffnungen verfügen und einen verlagerbaren Verschluss, der einen Zugriff nur auf entweder die eine oder die andere Öffnung zulässt. Mit zwei Öffnungen lässt sich die Kapazität der Schleuseneinrichtung vorteilhaft verdoppeln. Damit kann die doppelte Materialmenge eingeschleust oder ausgeschleust oder parallel ein- und ausgeschleust werden. Sollte jedoch eine der beiden Öffnungen für das Einschleusen und die andere Öffnung für das Ausschleusen von Material verwendet werden, so lässt sich ein Kontakt von sterilem und kontaminiertem Material zuverlässig vermeiden. Dafür sorgt der verlagerbare Verschluss, der zwingend entweder die eine oder die andere Öffnung verschließt. Er kann beispielsweise als klappbarer Deckel ausgebildet sein, der einen Teil der Arbeitsplatte darstellt. Verschließt er also eine der beiden Öffnungen, dient er als Teil der Arbeitsplatte. Alternativ kann er in der Ebene der Arbeitsplatte verschiebbar angeordnet sein, um entweder die eine oder die andere Öffnung freizugeben, jedenfalls aber nicht beide Öffnungen gleichzeitig.

An der Schleuseneinrichtung können Sicherheitsbehälter zur Aufnahme einer Anzahl von Behältern oder von losem Material luftdicht gekoppelt werden. Ihre Dimensionen und ihre Konstruktion können sich an den Bedingungen einer Autoklaviereinrichtung orientieren, in der sie samt Inhalt sterilisiert werden können, bevor sie an der Isolatoranordnung angekoppelt werden. Nach einer weiteren vorteilhaften Ausgestaltung der Erfindung können die Sicherheitsbehälter und die Schleusenvorrichtung über eine zusammenwirkende Verrastung der Sicherheitsbehälter an der Schleusenvorrichtung verfügen, die an den Verschluss der Schleusenvorrichtung derartig gekoppelt ist, dass nur eine aktivierte Verrastung eines Sicherheitsbehälters an der Schleusenvorrichtung eine Betätigung des Verschlusses ermöglicht. Ein derartiger Schleusensicherheitsverschluss stellt sicher, dass nicht durch eine Fehlbedienung eines Sicherheitsbehälters beim Ankoppeln an der Schleusenvorrichtung die sterile Atmosphäre in der Isolatoranordnung beeinträchtigt wird.

Nach einer weiteren vorteilhaften Ausgestaltung der Erfindung kann die Isolatoranordnung über eine Hubbeinrichtung für Behälter im Sicherheitsbehälter verfügen. Um eine optimale Platzausnutzung zu erreichen, können die Sicherheitsbehälter die vollständige Höhe vom Boden bis zu einer Unterseite der Arbeitsplatte einnehmen. Auf diese Weise können sie eine Vielzahl an Behältern übereinander aufnehmen. Die Hubeinrichtung hilft dem Benutzer dabei, die Behälter mit dem durch die Handschuheingriffe begrenzten Bewegungsspielraum und durch eine der beiden Öffnungen in der Arbeitsplatte hindurch aus den Sicherheitsbehältern zu entnehmen. Dazu braucht der Benutzer jeweils nur den obersten Behälter abzunehmen, weil die Hubeinrichtung die darunterliegenden Behälter anhebt und so auf ein Niveau in etwa unterhalb der Arbeitsplatte und damit in einen für den Benutzer leicht erreichbaren Bereich anhebt. Dadurch wird es möglich, die großvolumigen Sicherheitsbehälter unterhalb der Arbeitsplatte der oben beschriebenen Schleuseneinrichtung sinnvoll zu verwenden. Die Hubvorrichtung kann beispielsweise auf Federkraft beruhen oder auf einem Luftkissen unter den Behältern, das der Benutzer der Isolatoranordnung über einen Schlauch an eine Druckluftquelle anschließt.

Die Arbeitsplatte steht horizontal vor dem Regal und damit radial vom zylindrischen Regal ab. Nach einer weiteren vorteilhaften Ausgestaltung der Isolatoranordnung kann die Arbeitsplatte aus ihrer horizontalen Ebene heraus auf- oder abklappbar ausgebildet sein. Ist sie nicht in Betrieb, so kann sie den durch sie sonst eingenommenen Raum an dem Regal bzw. an der Isolatoranordnung freigeben, sodass die unbenutzte Isolatoranordnung weniger Platzbedarf aufweist. Sie kann vollständig zum Regal hin oder nur teilweise nach rechts oder links auf sich selbst geklappt werden. Das Klappen der Arbeitsplatte kann durch einen flexiblen oder abnehmbaren Abschnitt des Zelts ermöglicht werden, der die Handschuheingriffe umfasst. Dadurch kann es möglich sein, beispielsweise zwei Isolatoranordnungen nebeneinander und mit aufeinander zuweisenden Arbeitsplatten anzuordnen, wobei nur eine Isolatoranordnung mit abgeklappter Arbeitsplatte in Betrieb sein kann. Auf diese Weise lassen sich mehr erfindungsgemäße Isolatoranordnungen auf geringerem Raum unterbringen, womit sie insbesondere in kleineren Forschungseinrichtungen Verwendung finden können.

Die Arbeit an oder in einer Isolatoranordnung ist durch die Handschuheingriffe auch hinsichtlich des Bewegungsspielraums erschwert. Jede Erleichterung kann zu einer geringeren körperlichen Belastung und zu einer geringeren Fehlerquote beitragen. Nach einer weiteren vorteilhaften Ausgestaltung der Isolatoranordnung kann sie daher über Bedienungseinrichtungen zur Bedienung des Regals nach einem der obigen Ansprüche und/oder zur Bedienung der Steuerungseinrichtung der Isolatoranordnung nach Anspruch 6 für Benutzer verfügen, insbesondere per Keypad an einem Handschuheingriff und/oder über Spracherkennung und/per Fußtasten. Details zu diesen Vorrichtungen sind der Anmeldung WO 2011/124 209 A zu entnehmen, deren diesbezüglicher Inhalt auch zum Offenbarungsgehalt der vorliegenden Anmeldung gemacht wird.

Nach einer weiteren vorteilhaften Ausgestaltung der Erfindung kann die Isolatoranordnung über allseitig geschlossene Behälter mit Mikrofiltern an radial einander gegenüberliegenden Behälterseiten und ein Gebläse am Isolator zum zentralen Absaugen von Luft aus dem Zelt in die Umgebung verfügen. In dem geschlossenen Behälter kann leichter Unterdruck herrschen, da über das Gebläse Luft durch den Käfig gesaugt wird. Damit der Unterdruck im Behälter nicht zu stark wird, kann ein weiteres Gebläse gefilterte Luft in die Hülle blasen.

Dadurch lässt sich der Druck im Behälter durch gleichzeitiges Blasen und Saugen genau einstellen und regulieren. Die Inhalte dieser Behälter, beispielsweise Tiere in Käfigen, sind dann nicht nur olfaktorisch voneinander getrennt, was Wechselwirkungen zwischen den Tieren unterschiedlicher Behälter ausschließt. Auch eine gegenseitige Kontamination ist ausgeschlossen, was bei herkömmlichen Tierisolatoren nicht zwingend der Fall ist. Zudem wird die Luft zweimal gefiltert, das erste Mal beim Einströmen in die Hülle und ein weiteres Mal beim Einströmen in den Behälter. Dadurch lassen sich besonders zuverlässig eine hohe Luftqualität und die maximale Hygiene für Mensch und Tiere bei Haltung sicherstellen. Als Behälter kommen zum Beispiel tortenförmige Behälter in Frage wie sie von Animal Care Systems Inc. (www.animalcaresystems.com), oder von Braintree Scientific, Inc. (www.braintreesci.com/prodinfo.asp?number=MPC) erhältlich sind.

Das Prinzip der Erfindung wird im Folgenden anhand einer Zeichnung beispielhaft noch einmal erläutert. In der Zeichnung zeigen:
- Figur 1:: eine erste perspektivische Ansicht der Isolatoranordnung,
- Figur 2:: eine zweite perspektivische Ansicht derselben Isolatoranordnung,
- Figur 3:: eine perspektivische Ansicht einer weiteren Ausführungsform der Isolatoranordnung,
- Figur 4:: eine Dreh- und Hubvorrichtung der Isolatoranordnung,
- Figur 5:: eine mehrteilige Hülle der Isolatoranordnung und
- Figur 6:: eine Hubeinrichtung.

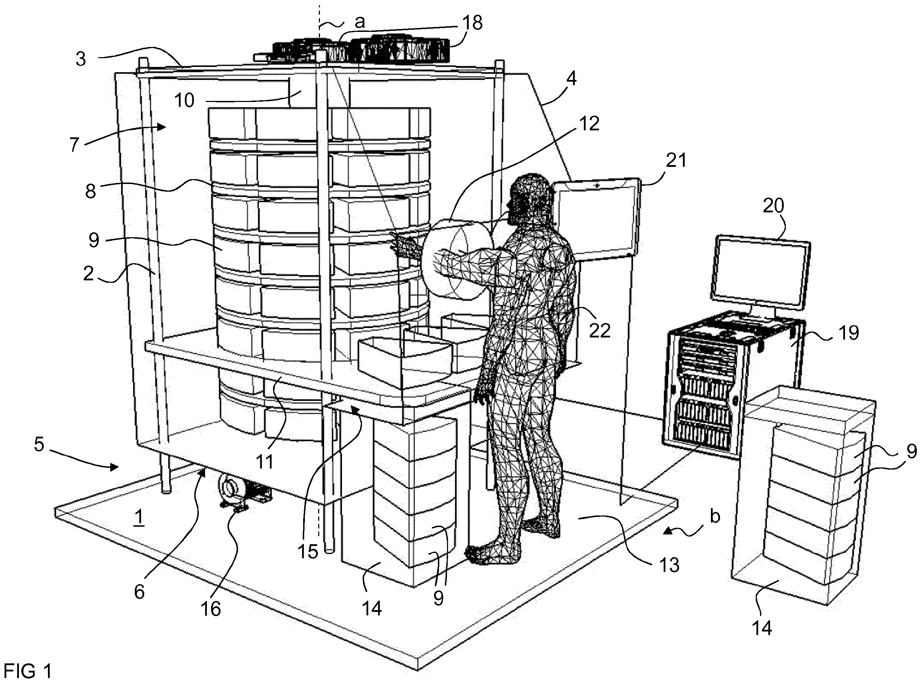

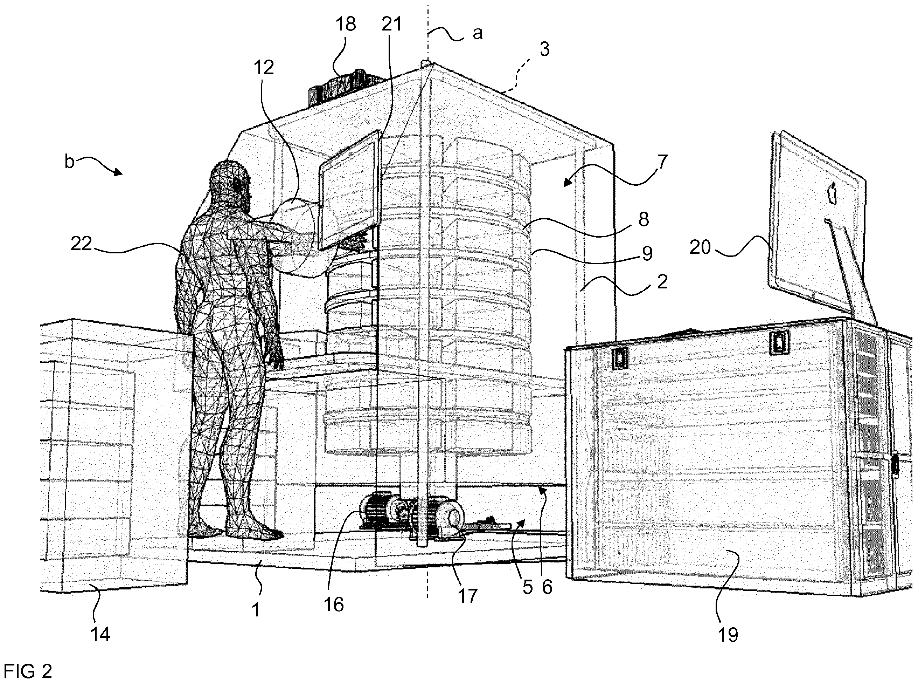

Figuren 1 und 2 zeigen perspektivische Ansichten derselben Isolatoranordnung, jeweils von einer Bedienungsseite b aus gesehen. Von einer Fußplatte 1 aus erheben sich vier Stützen 2, die eine Deckplatte 3 und eine kubische transparente Hülle 4 tragen. Die Hülle 4 reicht nicht ganz bis zur Fußplatte 1 herunter, sondern lässt einen Zwischenraum 5 zwischen ihrer Unterseite 6 und der Fußplatte 1 frei. In ihrem Innern nimmt die Hülle 4 ein zylindrisches Regal 7 mit einer Hochachse a auf, das auf acht scheibenförmigen Regaltellern 8 jeweils zehn kreissektorförmige Käfige 9 trägt. Die Regalteller 8 werden von einer zentrischen Mittelsäule 10 auf der Hochachse a des Regals 7 getragen. Die Mittelsäule 10 ist hohl und sowohl drehbar als auch entlang der Hochachse a vertikal verschiebbar.

Alternativ kann eine hohle Mittelsäule jeweils aus axialen Rohrabschnitten an den Regaltellern etwa in der Höhe eines Käfigs ausgebildet sein. Sie Rohrabschnitte können zumindest abschnittsweise konisch ausgebildet sein, so dass sie sich haftschlüssig ineinander stecken lassen. Sie können zusätzlich Luftanschlüsse für die Be- oder Entlüftung der Käfige enthalten. Sind die Regalteller zusätzlich untereinander drehbar gekoppelt, lassen sie sich unabhängig voneinander und ein einzelner Regalteller unabhängig vom übrigen Regal verdrehen.

Die Stützen 2 tragen innerhalb der Hülle 4 außerdem eine Arbeitsplatte 11, die auf der Benutzerseite b der Isolatoranordnung einseitig auskragt. Sie ist geringfügig breiter als der Durchmesser des zylindrischen Regals 7 und bietet einen Arbeitsraum von mindestens der Tiefe eines Käfigs 9. Drei Käfige 9 können auf ihr bequem abgestellt und auf ihr und innerhalb der Hülle 4 manipuliert werden. Dazu weist die Hülle 4 über der Arbeitsplatte 11 zwei Handschuheingriffe 12 auf, die innerhalb der Hülle 4 einen Zugriff auf die den Handschuheingriffen 12 zugewandten Käfige 9 im Regal 7 und auf die Arbeitsplatte 11 bietet.

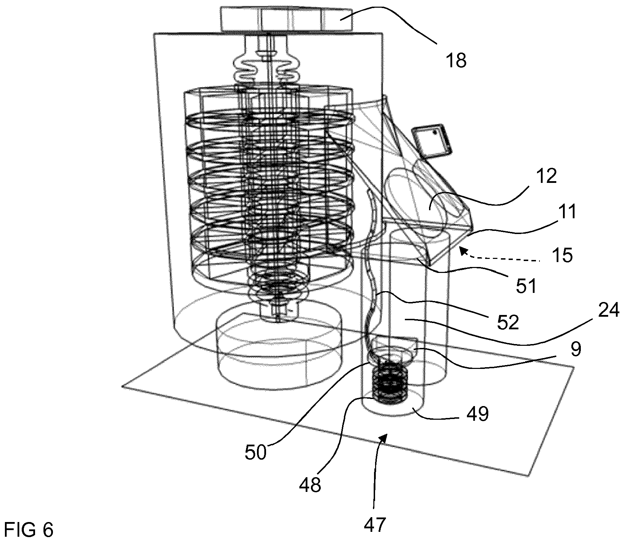

Unter der Arbeitsplatte 11 springt die Hülle 4 in Richtung des Regals 7 zurück und lässt damit einen Fußraum 13 frei. Er erstreckt sich unter der Auskragung der Arbeitsplatte 11 und reicht damit von ihrer dem Benutzer 22 zugewandten Vorderkante bis zu den ersten zwei Stützen 2 vor dem zylindrischen Regal 7. In den Fußraum 13 lassen sich kubische Sicherheitsbehälter 14 einfahren und an einer nicht näher dargestellten Schleuseneinrichtung 15 von unten an der Arbeitsplatte 11 luftdicht ankoppeln. Die Sicherheitsbehälter 14 können fünf Käfige 9 aufnehmen und lassen sich zusammen mit ihrem Inhalt sterilisieren. Über die Schleuseneinrichtung 15 lassen sie sich durch die geöffnete, zum Beispiel teilweise aufgeklappte oder aufgeschobene Arbeitsplatte 11 hindurch betätigen, nämlich entnehmen und auf die verbleibende Arbeitsplatte 11 stellen. Damit sie noch im Sicherheitsbehälter 14 von den Handschuheingriffen 12 aus bedient werden können und erreichbar sind, verfügen die Sicherheitsbehälter 14 über eine in Figur 6 dargestellte Hubeinrichtung zum Anheben der Käfige 9 in etwa auf das Niveau der Arbeitsplatte 11.

Damit auch die Käfige 9 der obersten und der untersten Regalteller 8 und die vom Benutzer 22 aus gesehen hintersten Käfige 9 von den Handschuheingriffen 12 aus erreicht werden können, lassen sich die Regalteller 8 und mit ihnen das zylindrische Regal 7 verdrehen und vertikal verfahren. Dazu verfügt die Isolatoranordnung im Zwischenraum 5 über einen Hubmotor 16 und einen Drehmotor 17 (Figur 2). Sie sind außerhalb der Hülle 4 angeordnet und können daher die sterile und keimfreie Atmosphäre innerhalb der Hülle 4 nicht beeinträchtigen. Zudem sind sie für Wartungs- und Reparaturarbeiten problemlos zu erreichen, ohne dass der Innenraum der Hülle 4 dadurch beeinträchtigt wird.

Auf der Oberseite der Isolatoranordnung und dort auf der Deckplatte 3 sind ein erstes und ein zweites Gebläse 18 angeordnet, die über einen Filter Luft in die Hülle 4 einblasen und durch die hohle Mittelsäule 10 hindurch wieder absaugen. Die Gebläse 18 erlauben, den Druck in den Käfigen 9 genau zu regulieren. Denn die Käfige 9 sind geschlossen ausgebildet und verfügen über einen (nicht dargestellten) Deckel sowie an ihrer dem Benutzer 22 zugewandten gekrümmten Vorderseite und an ihrer schmalen, der Mittelsäule 10 zugewandten Rückseite jeweils einen (nicht dargestellten) Luftdurchgang mit einem Mikrofilter. Sobald die Mittelsäue 10 über das erste Gebläse 18 Luft absaugt, entsteht im Käfig 9 ein Unterdruck. Er lässt sich regulieren, indem das zweite Gebläse 18 gefilterte Luft in die Hülle 4 bläst. So lässt sich der Druck in den Käfigen 9 durch Blasen und Saugen genau regulieren. Zudem sind Tiere in den gedeckelten Käfigen 9 olfaktorisch getrennt, was bei gewöhnlichen Tierisolatoren in der Regel nicht der Fall ist. Schließlich wird die Luft mehrmals gefiltert: Das erste Mal beim Einströmen in die Hülle 4 und ein weiteres Mal beim Einströmen in die Käfige 9. Selbst die ggf. belastete Luft aus den Käfigen 9 wird beim Austritt aus den Käfigen 9 durch einen Mikrofilter an deren Rückseite gefiltert. Damit lassen sich hohe hygienische Vorstellungen auch zum Schutz des Bedieners 22 der Isolatoranordnung erfüllen.

Je nach Anwendung der Isolatoren können sie mit Über- oder Unterdruck betrieben werden, bei Infektionskrankheiten zum Schutz des Benutzers 22 in der Regel mit Unterdruck, bei gnotobiotischen Anwendungen Schutz des Tieres mit Überdruck. Überdruck und Unterdruck lassen sich durch eine entsprechende Ansteuerung der Gebläse 18 einstellen. Außerdem lässt sich durch Ansteuerung der Gebläse 18 auch der Luftstrom durch die Käfige 9 hindurch umkehren. Dazu wird Luft durch die Mittelsäule 10 hindurch in die Käfige 9 geblasen und von dort über den übrigen Innenraum der Hülle 4 abgesaugt.

Die Isolatoranordnung verfügt außerdem über einen Rechner 19 mit einem Bildschirm 20, auf dem eine Steuerungseinrichtung für die und ggf. weitere (nicht dargestellte) Isolatoranordnungen und eine Datenbank mit Daten über den Inhalt der Käfige 9 und ggf. weiterer Käfige enthalten ist. Für einen schnelleren Zugriff bzw. Blick auf den Rechner 19 verfügt die Isolatoranordnung außerdem außenseitig über einen Bildschirm 21 rechts neben den Handschuheingriffen 12, der berührsensibel ist (Touchscreen). Zusätzlich trägt der Benutzer 22 einen kleinen, am Unterarm verschnallten (nicht dargestellten) Touchscreen oder Keypad, den bzw. das er durch die Handschuheingriffe 12 hindurch während der Arbeit an den Käfigen 9 betätigen kann, ohne die Handschuheingriffe 12 abstreifen zu müssen.

Der Benutzer 22 der Isolatoranordnung kann zunächst am Rechner 19 die Datenbank über die Käfiginhalte einsehen, sich am Bildschirm 21 als Benutzer anmelden und dergleichen. Tätigkeiten in der Isolatoranordnung führt er aus, indem er die Handschuheingriffe 12 betätigt. Jetzt kann er innerhalb der Hülle 4 Leistungen an den Käfigen 9 oder deren Inhalt erbringen oder Tätigkeiten vornehmen. Dazu kann er einzelne Käfige 9 auf der Arbeitsplatte 11 abstellen und sie bearbeiten. Da die Handschuheingriffe 12 nur eine beschränkte Bewegungsmöglichkeit erlauben und sich die Käfige 9 der oberen und der unteren Regalteller 8 nicht ohne Unterstützung erreichen lassen, kann der Benutzer 22 das Regal per Fußtastensteuerung oder per Eingabe am Keypad motorisch drehen und anheben bzw. absenken. So lässt sich jeder Käfig 9 in die Reichweite der Handschuheingriffe 12 bringen.

Neue Käfige 9 oder neues Material gelangen über den Sicherheitsbehälter 14 in die Isolatoranordnung. Nach ihrem Ankoppeln an der Schleuseneinrichtung 15 lassen sich frische Käfige 9 aus dem Sicherheitsbehälter 14 gegebenenfalls per Hubeinrichtung auf die Arbeitsplatte 11 stellen und dort weiter bearbeiten. Kontaminiertes Material bzw. verschmutzte Käfige 9 lassen sich in umgekehrter Weise über den zweiten Sicherheitsbehälter 14 ausschleusen. Am Bildschirm 21 oder - ohne Unterbrechung seiner Arbeiten - am Keypad kann der Benutzer 22 seine Vorgänge und Leistungen anmelden und/oder vorgeschlagene und durchgeführte Vorgänge und Leistungen protokollieren, worauf hin die Datensätzen in der Datenbank aktualisiert werden.

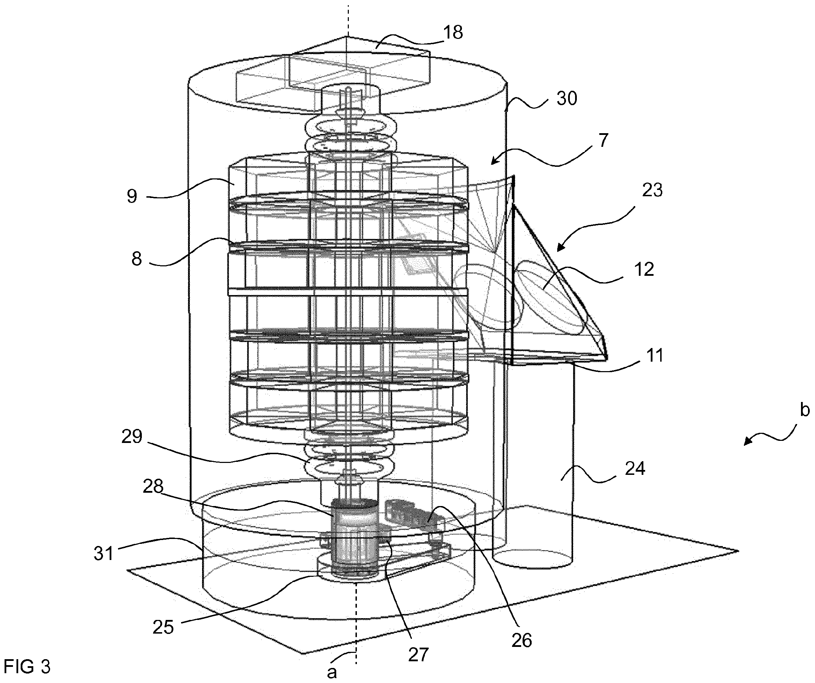

Figur 3 zeigt eine weitere Ausgestaltungsform einer erfindungsgemäßen Isolatoranordnung. Sie ist prinzipiell vergleichbar mit derjenigen der Figuren 1 und 2 aufgebaut, nämlich mit einem zylindrischen Regal 7, das sechs Regalteller 8 mit jeweils acht Käfigen 9 umfasst. Das zylindrische Regal 7 ist von einer zylindrischen Hülle 30 umgeben, an deren Benutzerseite b der Hülle 30 eine Arbeitsplatte 11 auskragt. In einem in einer Seitenansicht dreieckigen Arbeitsbereich über der Arbeitsplatte 11 sind zwei Handschuheingriffe 12 untergebracht. Unter der Arbeitsplatte 11 lassen sich zylindrische Sicherheitsbehälter 24 in der oben geschriebenen Weise verrasten und betätigen. Auf ihrer Oberseite trägt die Isolatoranordnung zwei Gebläse 18, die den Innenraum der zylindrischen Hülle 30 und die Käfige 9 in der oben beschriebenen Weise mit Frischluft versorgen und verbrauchte Luft absaugen.

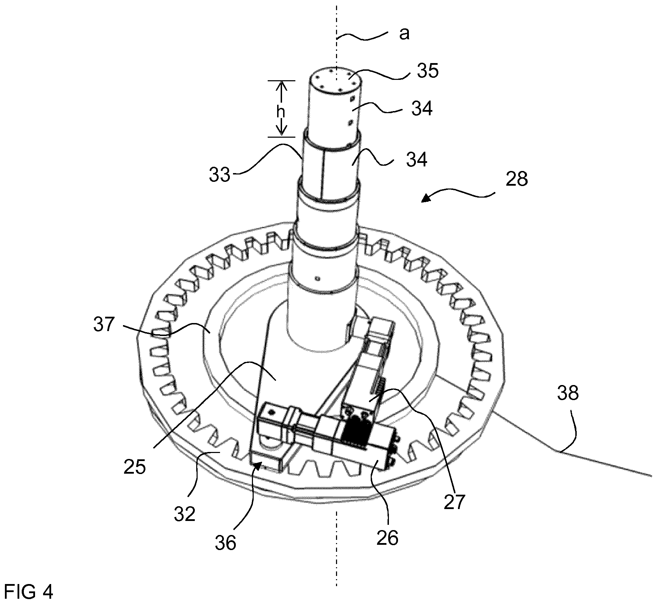

Die Isolatoranordnung der Figur 3 verfügt abweichend über einen zylindrischen und abnehmbaren Tragering 31 mit einem etwas geringeren Durchmesser als die Hülle 30. Auf ihm ruht die Hülle 30. Der Tragering 31 umgibt einen Rotationsschenkel 25, der von einem Drehmotor 26 angetrieben und in der in Figur 4 detaillierter erläuterten Weise in Rotation versetzt wird. Auf dem Rotationsschenkel 25 ist ein teilweise verdeckter Hubmotor 27 angeordnet, der eine Verdrehung des Rotationsschenkels 25 über den Drehmotor 26 mitmacht. Der Hubmotor 27 verfügt über eine Spindelhubmechanik 28, die das Regal 7 entlang einer Hochachse auf- und abwärts verfährt. Sowohl der Hubmotor 27 als auch die Spindelhubmechanik 28 sind außerhalb der feststehenden Hülle 30 angeordnet. Während der Hubmotor 27 unterhalb der Hülle 30 positioniert ist, ragt die Spindelhubmechanik 28 von einem Balg 29 ummantelt in die Hülle 30 hinein. Der Balg 29 stellt quasi einen vertikal verformbaren, aber drehfesten Abschnitt der stationären zylindrischen Hülle 30 dar, der einen luftdichten Übergang zwischen dem Innenraum der Hülle 30 und den von außen am umhüllten zylindrischen Regal 7 angreifenden Dreh- und Hubkräften schafft. Damit bleibt einerseits der Inhalt der zylindrischen Hülle 30 und insbesondere das zylindrischen Regal 7 luftdicht und keimfrei gegenüber der Umgebung abgeschlossen. Andererseits können der Hubmotor 27 und Drehmotor 26 von einer Außenseite der zylindrischen Hülle 30 aus gewartet und gegebenenfalls repariert werden, ohne die hygienischen Verhältnisse innerhalb der zylindrischen Hülle 30 zu beeinträchtigen.

Figur 4 zeigt den vertikalen und rotatorischen Antrieb des Regals 7 im Detail: der Drehmotor 26 und der Hubmotor 27 sitzen gemeinsam auf dem Rotationsschenkel 25. Er rotiert um die Hochachse a und erstreckt sich radial bis zu einem innen verzahnten Zahnkranz 32, der koaxial zur Hochachse a angeordnet ist. Ebenfalls koaxial ist die Spindelhubmechanik 28 auf dem Rotationsschenkel 25 angebracht, die sich vom Hubmotor 27 antreiben lässt. Sie umfasst einen teleskopierbare Hubstange 33 aus fünf zylindrischen Segmenten 34 der Höhe h. Die Hubstange 33 lässt sich damit auf etwa die fünffache Höhe h eines Segments 34 ausfahren. Auf dem oberseitigen Ende der Hubstange 33 ist eine drehbare Scheibe 35 als Teil einer nicht dargestellten Drehdichtung des feststehenden Balgs 29 (Figur 3) gegenüber dem drehbaren Regal 7 angeordnet. Die Scheibe 35 lässt sich gegenüber dem Balgs 29 verdrehen, dichtet aber die Atmosphäre innerhalb der Hülle 30 (Figur 3) gegenüber der Umgebung hermetisch ab.

Der Rotationsschenkel 25 greift an seinem radial äußeren Ende 36 über ein verdecktes Zahnrad in den Zahnkranz 32 ein. Der Drehmotor 26 treibt das Zahnrad an und versetzt den Rotationsschenkel 25 und mit ihm sich selbst, den Hubmotor 27 und die Spindelhubmechanik 28 in eine Rotation. Der Rotationsschenkel 25 überstreicht dabei einen koaxialen Schleifring 37, der mit verdeckt angeordneten Schleifkontakten an einer Unterseite des Rotationsschenkels 25 zusammenwirkt. Der Schleifring 37 ist über eine Stromleitung 38 an eine herkömmliche Stromversorgung angeschlossen und versorgt sowohl den Drehmotor 26 als auch den Hubmotor 27 mit Energie.

Damit ergibt sich ein Antrieb für die vertikale und die rotatorische Bewegung des Regals 7, der eine äußerst geringe Bauhöhe beansprucht und damit vorteilhaft unter dem Regal 7 platziert werden kann, ohne ihm für Käfige 9 nutzbaren Stauraum zu nehmen. Zugleich lassen sich die Motoren 26, 27 außerhalb der Hülle 30 (Figur 3) anordnen, womit sie für Wartungs- und Reparaturarbeiten leicht zugänglich sind, und die Atmosphäre innerhalb der Hülle 30 weder dabei noch durch ihren bestimmungsgemäßen Betrieb beeinträchtigt wird.

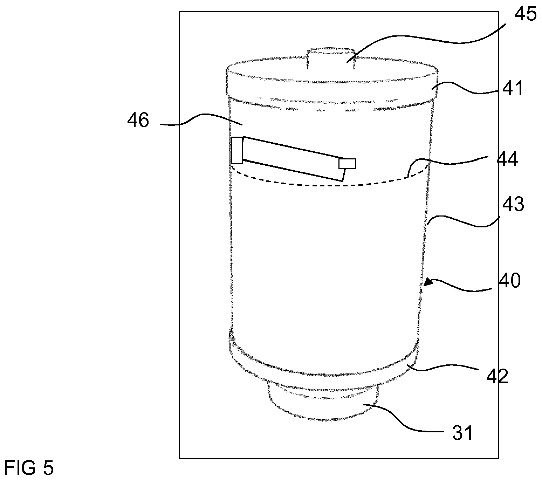

Figur 5 ist eine schematische Widergabe einer mehrteiligen zylindrischen Hülle 40. Sie setzt sich aus einem tellerförmigen Deckel 41, einem ebensolchen Boden 42 und einem transparenten Zylindermantel 43 zusammen, der durch Zwischenringe 44 in vertikaler Richtung in einzelne Mantelabschnitte 46 unterteilt sein kann. Der Deckel 41 weist einen kragenförmigen Ansatz 45 als Anschluss an die Gebläse 18 (Figur 3) auf. Der Boden 42 stützt die Hülle 40 auf dem Tragering 31 (Figur 3) ab. Die Zwischenringe 44 erlauben ein Zerlegen des Mantels 43 in mehrere Mantelabschnitte 46, so dass die Hülle 40 zum Beispiel an eine veränderte Höhe des Regals 7 angepasst werden kann. Außerdem ermöglichen sie den Austausch beschädigter Mantelabschnitte 46, ohne dass der Mantel 43 vollständig ersetzt werden müsste. Schließlich lässt sich die Höhe der Handschuhengriffe 12 (Figur 3), die an einem speziellen nicht dargestellten Mantelabschnitt angebracht sind, durch Einfügen oder Entnahme einzelner Mantelabschnitte 46 an veränderte Gegebenheiten anpassen. Ein Abdichten verschiedener Mantelabschnitte 46 lässt sich durch Überkleben der Fugen zwischen den Mantelanschnitten 46 und den Zwischenringe 45 mit transparentem Klebeband erreichen.

Figur 6 zeigt zusätzlich zu der in der in Figur 3 gezeigten Isolatoranordung eine dort nicht dargestellte Hubvorrichtung 47 in den Sicherheitsbehältern 24. Sie umfasst ein durch Aufblasen balgartig expandierbares zylindrisches Luftkissen 48, das auf einem Behälterboden 49 des Sicherheitsbehälters 24 ruht. Das Luftkissen 48 trägt eine scheibenförmige Platte 50, auf der ein Käfig 9 abgestellt ist. Durch die Platte 50 hindurch und über eine Oberseite 51 des Sicherheitsbehälters 24 hinaus verläuft ein Luftschlauch 52, der über nicht dargestellte Kupplungen und Ventile an eine ebenfalls nicht dargestellte Luftleitung aus dem Gebläse 18 als Druckluftquelle anschließbar ist.

Sobald der Sicherheitsbehälter 24 unter der Arbeitsplatte 11 an die dortige Schleuseneinrichtung 25 luftdicht angekoppelt ist, kann der Benutzer 22 (Figuren 1 bis 3) den Sicherheitsbehältern 24 öffnen. Ist er maximal gefüllt, kann er einen obersten Käfig 9 entnehmen. Befindet sich aber nur ein Käfig 9, reicht seine Bewegungsmöglichkeit in den Handschuheingriffen 12 nicht aus, bis in den Sicherheitsbehälter 24 hineinzugreifen. Daher schließt er den im Sicherheitsbehälter 24 im Bereich seiner Oberseite 51 bereitliegenden Luftschlauch 52 an die Gebläse 18 als Druckluftquelle an. Sie expandieren daraufhin das Luftkissen 48, das den Käfig 9 vertikal anhebt, bis ihn der Benutzer 22 entnehmen kann.

### Bezugszeichenliste:

- 1: Fußplatte
- 2: Stütze
- 3: Deckplatte
- 4: Hülle
- 5: Zwischenraum
- 6: Unterseite
- 7: zylindrisches Regal
- 8: Regalteller
- 9: Käfig
- 10: Mittelsäule
- 11: Arbeitsplatte
- 12: Handschuheingriff
- 13: Fußraum
- 14: Sicherheitsbehälter
- 15: Schleuseneinrichtung
- 16: Hubmotor
- 17: Drehmotor
- 18: Gebläse
- 19: Rechner
- 20: Bildschirm
- 21: Bildschirm (Touchscreen)
- 22: Benutzer
- 23: Arbeitsbereich
- 24: Sicherheitsbehälter
- 25: Rotationsschenkel
- 26: Drehmotor
- 27: Hubmotor
- 28: Spindelhubmechanik
- 29: Balg
- 30: zylindrische Hülle
- 31: Tragering
- 32: Zahnkranz
- 33: Hubstange
- 34: Segment
- 35: Scheibe
- 36: äußeres Ende
- 37: Schleifring
- 38: Stromleitung
- 40: Hülle
- 41: Deckel
- 42: Boden
- 43: Mantel
- 44: Zwischenring
- 45: Ansatz
- 46: Mantelabschnitt
- 47: Hubvorrichtung
- 48: Luftkissen
- 49: Boden des Sicherheitsbehälter 24
- 50: Platte
- 51: Oberseite des Sicherheitsbehälter 24
- 52: Luftschlauch

- a: Hochachse
- b: Benutzerseite
- h: Höhe eines Segments 34

## Patentansprüche

1. Isolatoranordnung zum Herstellen und zum Aufrechterhalten einer keimfreien Umgebung, insbesondere für die Pflanzenzucht oder die Tierforschung oder die Vorbereitung von Medikamenten, mit
- einem zylindrischen Regal mit einer Hochachse,
- im Regal umfangsseitig angeordneten und in radialer Richtung zugänglichen Behältern ,
- einer Arbeitsplatte in unmittelbarer räumlicher Nähe zu dem Regal mit einem darunter/davor liegenden Fußbereich,
- einem Zelt zur gemeinsamen sterilen Abschirmung des Regals und zugleich der Arbeitsplatte gegenüber der Umgebung,
- mit Handschuheingriffen im Zelt zur Bedienung und/oder Bearbeitung der Behälter und/oder ihres Inhalts zwischen dem Regal und der Arbeitsplatte,
- einer Schleuseneinrichtung im Zelt zum Einbringen sterilen Materials in das Zelt und zum Entnehmen kontaminierten Materials aus dem Zelt,
- einer Vorrichtung zur Verlagerung des Regals relativ zur Arbeitsplatte in einer axialen Richtung und in einer rotatorischen Richtung.

2. Isolatoranordnung nach Anspruch 1, **gekennzeichnet durch** (jeweils) einen motorischen Antrieb zur Verlagerung des Regals relativ zur Arbeitsplatte vorzugsweise außerhalb des Zelts.

3. Isolatoranordnung nach Anspruch 1 oder 2, **dadurch gekennzeichnet, dass** das Regal einzelne horizontal verlaufende scheibenförmige Teller umfasst, auf denen sich die Behälter abstellen lassen.

4. Isolatoranordnung nach einem der obigen Ansprüche, **gekennzeichnet durch** einen Spindelhubantrieb oder einen Scherenhubantrieb als Vorrichtung zur axialen Verlagerung.

5. Isolatoranordnung nach einem der obigen Ansprüche, **gekennzeichnet durch** eine drehbare Vorrichtung zur axialen Verlagerung des Regals relativ zur Arbeitsplatte.

6. Isolatoranordnung nach einem der obigen Ansprüche, **gekennzeichnet durch** einen mehrteiligen Aufbau des Zelts aus miteinander koppelbaren Abschnitten.

7. Isolatoranordnung nach einem der obigen Ansprüche, **gekennzeichnet durch** eine Steuerungseinrichtung zur Steuerung der Bewegungen des Regals und/oder zur Steuerungseinrichtung von Vorgängen mit Behältern und/oder Leistungen an Objekten in den Behältern und eine Datenbank zur Erfassung der Objekte und zur Dokumentation von Vorgängen mit und Leistungen an Behältern und/oder deren Objekten.

8. Isolatoranordnung nach einem der obigen Ansprüche, **gekennzeichnet durch** Kennzeichnungen der Behälter zu deren Individualisierung und durch korrespondierende Leseeinrichtungen an der Isolatoranordnung.

9. Isolatoranordnung nach einem der obigen Ansprüche, **gekennzeichnet durch** eine in der Arbeitsplatte integrierte Schleuseneinrichtung.

10. Isolatoranordnung nach dem obigem Anspruch, **gekennzeichnet durch** zwei Öffnungen in der Arbeitsplatte und einen verlagerbaren Verschluss, der einen Zugriff auf entweder die eine oder die andere Öffnungen zulässt.

11. Isolatoranordnung nach dem obigem Anspruch, **gekennzeichnet durch** eine Verrastung der Sicherheitsbehälter an der Schleusenvorrichtung und durch eine Koppelung der Verrastung an den Verschluss der Schleuseneinrichtung, sodass nur eine aktivierte Verrastung eine Betätigung des Verschlusses ermöglicht.

12. Isolatoranordnung nach dem obigen Anspruch, **gekennzeichnet durch** eine Hubeinrichtung für Käfige im Sicherheitsbehälter.

13. Isolatoranordnung nach einem der obigen Ansprüche, **dadurch gekennzeichnet, dass** die Arbeitsplatte aus einer horizontalen Ebene heraus auf- oder abklappbar ausgebildet ist.

14. Isolatoranordnung, **gekennzeichnet durch** Bedienungseinrichtungen zur Bedienung des Regals nach einem der obigen Ansprüche und/oder zur Bedienung der Steuerungseinrichtung der Isolatoranordnung nach einem der Ansprüche 7 bis 13 für Benutzer per KeyPad an einem Handschuheingriff und/oder über Spracherkennung und/oder per Fußtasten.

15. Isolatoranordnung nach einem der obigen Ansprüche, **gekennzeichnet durch** allseitig geschlossene Behälter mit Mikrofiltern an radial einander gegenüberliegenden Behälterseiten und einem Gebläse zum zentralen Absaugen von Luft aus dem Zelt in die Umgebung.

## Claims

1. Isolator assembly for producing and maintaining a sterile environment, in particular for plant cultivation or animal research or preparation of medicaments comprising
- a cylindrical rack with a vertical axis,
- containers arranged circumferentially in the rack and accessible in a radial direction,
- a worktop in the immediate vicinity of the rack with a foot region situated underneath/in front of the worktop,
- a tent for the joint sterile shielding of both the rack and at the same time the worktop with respect to the surrounding area,
- with gloved intervention points in the tent for operating and/or processing the containers and/or the contents thereof between the rack and the worktop,
- an air lock device in the tent for introducing sterile material into the tent and for removing contaminated material from the tent,
- a device for repositioning the rack relative to the worktop in an axial direction and in a rotational direction.

2. The isolator assembly according to claim 1, **characterized by** (in each case) a motor drive for repositioning the rack relative to the worktop preferably outside the tent.

3. The isolator assembly according to claim 1 or 2, **characterized in that** the rack comprises individual horizontally running disk-shaped plates on which the containers can be placed.

4. The isolator assembly according to one of the above claims, **characterized by** a spindle elevating drive or a scissors elevating drive as the device for axial repositioning.

5. The isolator assembly according to one of the above claims, **characterized by** a rotatable device for the axial repositioning of the rack relative to the worktop.

6. The isolator assembly according to one of the above claims, **characterized by** a multi-part structure of the tent from sections which can be coupled together.

7. The isolator assembly according to one of the above claims, **characterized by** a control device for controlling the movements of the rack and/or for controlling processes with containers and/or work on objects in the containers and a database for recording the objects and for documenting processes with and work on containers and/or their objects.

8. The isolator assembly according to one of the above claims, **characterized by** identifications of the containers for the individualization thereof and by corresponding reading devices on the isolator assembly.

9. The isolator assembly according to one of the above claims, **characterized by** an air lock device integrated in the worktop.

10. The isolator assembly according to the above claim, **characterized by** two openings in the worktop and a repositionable closure which allows access to either the one or the other openings.

11. The isolator assembly according to the above claim, **characterized by** a latching mechanism of the safety container on the air lock device and by a coupling of the latching mechanism to the closure of the air lock device so that only an activated latching mechanism allows an actuation of the closure.

12. The isolator assembly according to the above claim, **characterized by** a lifting device for cages in the safety container.

13. The isolator assembly according to one of the above claims, **characterized in that** the worktop is configured so that it can be folded out or folded down out from a horizontal plane.

14. isolator assembly, **characterized by** operating devices for operating a rack according to one of the above claims and/or for operating the control device of the isolator assembly according to one of claims 7 to 13 for users by keypad at a gloved intervention point and/or via voice recognition and/or by foot pedals.

15. The isolator assembly according to one of the above claims, **characterized by** containers closed on all sides with microfilters on radially opposite container sides and a fan for central extraction of air from the tent into the surrounding area.

## Revendications

1. Ensemble d'isolation pour la réalisation et le maintien d'un environnement stérile, en particulier dans le cadre de la phytogénétique ou de la recherche animale ou de la préparation de médicaments, avec
- un rayonnage cylindrique avec axe vertical,
- des conteneurs disposés en périphérie dans le rayonnage et accessibles en direction radiale,
- un plan de travail à proximité spatiale directe du rayonnage avec une zone pédestre située en dessous/devant
- une tente pour la protection commune stérile du rayonnage et aussi du plan de travail vis-à-vis de l'environnement,
- avec des systèmes à gants dans la tente pour la manipulation et/ou le traitement des conteneurs et/ou de leur contenu entre le rayonnage et la plan de travail,
- un dispositif à sas dans la tente pour y placer da la matière stérile dans la tente et pour retirer de la matière contaminée hors de la tente,
- un dispositif destiné à déplacer le rayonnage par rapport au plan de travail dans une direction axiale et dans une direction rotative.

2. Ensemble d'isolation selon la revendication 1, caractérisé (respectivement) par un mécanisme d'entraînement motorisé destiné à déplacer le rayonnage par rapport au plan de travail, de préférence, en dehors de la tente.

3. Ensemble d'isolation selon la revendication 1 ou 2, **caractérisé en ce que** le rayonnage comprend des plateaux en forme de disque passant horizontalement sur lesquels les conteneurs peuvent être déposés.

4. Ensemble d'isolation selon l'une quelconque des revendications précédentes, **caractérisé par** un mécanisme de levage à broche ou un mécanisme de levage articulé en tant que dispositif pour déplacement axial.

5. Ensemble d'isolation selon l'une quelconque des revendications précédentes, **caractérisé par** un dispositif pouvant tourner pour le déplacement axial du rayonnage par rapport au plan de travail.

6. Ensemble d'isolation selon l'une quelconque des revendications précédentes, **caractérisé par** une structure en plusieurs parties de la tente composée de sections pouvant être couplées les unes autres.

7. Ensemble d'isolation selon l'une quelconque des revendications précédentes, **caractérisé par** un dispositif de commande destiné à commander les mouvements du rayonnage et/ou pour commander des opérations avec des conteneurs et/ou des prestations sur des objets dans les conteneurs et par une banque de données destinée à la saisie des objets et pour la documentation des opérations et des prestations sur les conteneurs et/ou les objets de ceux-ci.

8. Ensemble d'isolation selon l'une quelconque des revendications précédentes, **caractérisé par** des identifications des conteneurs pour l'individualisation de ceux-ci et par des dispositifs de lecture correspondants sur l'ensemble d'isolation.

9. Ensemble d'isolation selon l'une quelconque des revendications précédentes, **caractérisé par** un dispositif à sas intégré dans le plan de travail.

10. Ensemble d'isolation selon la revendication précédente, **caractérisé par** deux ouvertures dans le plan de travail et une fermeture déplaçable, qui autorise un accès soit à l'une, soit à l'autres des ouvertures.

11. Ensemble d'isolation selon la revendication précédente, **caractérisé par** un enclenchement des conteneurs de sécurité sur le dispositif à sas et par un couplage de l'enclenchement à la fermeture du dispositif à sas de telle manière que seul un enclenchement activé permette un actionnement de la fermeture.

12. Ensemble d'isolation selon la revendication précédente, **caractérisé par** un dispositif de levage pour cages dans le conteneur de sécurité.

13. Ensemble d'isolation selon l'une quelconque des revendications précédentes, **caractérisé en ce que** le plan de travail est constitué d'un plan horizontal escamotable ou rabattable.

14. Ensemble d'isolation, **caractérisé par** des dispositifs de manutention pour la manutention du rayonnage, selon l'une quelconque des revendications précédentes et/ou pour la manutention du dispositif de commande de l'ensemble d'isolation selon l'une quelconque des revendications 7 à 13 pour l'utilisateur en fonction d'un clavier sur un système à gants et/ou par reconnaissance vocale et/ou par pédales.

15. Ensemble d'isolation selon l'une quelconque des revendications précédentes, **caractérisé par** des conteneurs fermés de tous côtés avec des microfiltres sur les côtés de conteneur radialement opposés les uns aux autres et une soufflerie pour l'extraction centrale d'air de la tente dans l'environnement.
